# EUROPEAN PATENT SPECIFICATION

(11) **EP 3 796 864 B1**
(45) Date of publication and mention of the grant of the patent: **15.04.2026**
(21) Application number: 19740054.2
(22) Date of filing: 22.05.2019
(51) Int. Cl.: A61B 34/00, A61B 34/20, A61B 90/20, A61F 9/007

(54) **SYSTEM UTILIZING SURGICAL TOOLING EQUIPMENT WITH GRAPHICAL USER INTERFACES**
SYSTEM ZUR VERWENDUNG VON CHIRURGIEINSTRUMENTEN MIT GRAFISCHEN BENUTZEROBERFLÄCHEN
SYSTÈME UTILISANT UN OUTIL CHIRURGICAL AVEC DES INTERFACES UTILISATEUR GRAPHIQUES

(30) Priority: 23.05.2018 US 201862675494 P
(43) Date of publication of application: 31.03.2021
(73) Proprietor: Alcon Inc., 1701 Fribourg (CH)
(72) Inventor: ZIEGER, Peter, 14513 Teltow (DE); GRUENDIG, Martin, 14513 Teltow (DE)
(74) Representative: Mooser, Sebastian Thomas
(86) International application number: PCT/IB2019/054241
(87) International publication number: WO 2019/224745

(56) References cited:
- WO-A1-02/100285
- WO-A1-2005/001679
- WO-A1-2016/182611
- US-A1- 2007 125 633
- US-A1- 2007 288 194
- US-A1- 2008 059 915
- US-A1- 2010 231 509
- US-B1- 8 169 404

## Description

### BACKGROUND

### Field of the Disclosure

This disclosure relates to quality assurance in medical procedures and more particularly to systems and methods for authenticating patient information with the medical procedure or process elements of the medical procedure.

### Description of the Related Art

Computer systems can assist surgeons in surgeries. The computer systems provide graphical user interfaces. However, in a sterile environment surgeons cannot easily touch non-sterile devices, such as interfaces to computer systems. Currently, surgeons have different possibilities to interact with interfaces to computer systems, such as foot pedals, a surgical assistant (e.g., medical personnel), and one-time disposals (e.g. Q-tips) to interact on a touch-screen and/or a keyboard. These solutions can be error prone and can lead to a wrong input. For example, during the interaction with a computer system, a surgeon may have to physically move his or her hand and/or head from a patient to a computer system interface to ensure that his or her computer system input is correct. This can be a potential distraction during a surgery, which can lead to unforeseen and/or negative surgical results.

Document WO 2016/182611 A1 discloses a surgical system used to enable a surgical tool as a control input in vitreoreinal surgery based on software tracking. The surgical system comprises an eyepiece, a microscope, a heads-up display configured in the surgical microscope, a control unit, a surgical tool, an imaging unit tracking a motion of the surgical tool and capturing at least one image of the surgical tool and a surgical site, and a processing unit processing the motion of the surgical tool to obtain a temporal spatial information of the surgical tool.
In document WO 02/100285 A1, a probe is described that is to be held by a user performing a surgical operation within a defined region while employing an image-based guide system having a display for displaying computer-generated images of a subject of an operation. The probe has a position which is tracked by the system and which is visible to the user, wherein, by moving the probe, the user is able to enter information into the system to cause changes in the physical shape of the subject in the image presented by the computer.

WO 2005/001679 A1 discloses a method for selecting a specific area of a display surface of a screen. The aim of the document is to provide a method of said kind which avoids the use of conventional input devices. For this purpose, the position and location of a pointer instrument in space is detected by means of a navigation system and defined orientations of the pointer instrument are associated with selected areas of the display surface.

US 2010/231509 A1 discloses a sterile networked interface system comprising a hand-held surgical tool and a data processing system. The surgical tool includes a sensor for sensing a physical variable related to the surgery, a wireless communication unit to transmit the physical variable to the data processing system, and a battery for powering the hand-held surgical tool. The surgical tool sends the physical variable and orientation information responsive to a touchless gesture control and predetermined orientation of the surgical tool.

### SUMMARY

The present invention concerns a system according to claim 1. The invention is defined by the subject-matter of the independent claim. Advantageous embodiments are outlined in the dependent claims.

In particular, a system is disclosed herein that is able to display a graphical user interface that includes at least one icon via a display; receive a first image from an image sensor; and determine, from the first image and a digital model of surgical tooling equipment, a first position of the surgical tooling equipment within the first image. For example, the surgical tooling equipment to be utilized as the pointer may include a scalpel, a Q-tip, tweezers, etc. The system may include or may be coupled to the display that displays the graphical user interface. The system may include a microscope integrated display that includes the display, which displays the graphical user interface. The system may further display a cursor of the graphical user interface at a second position associated with the first position. The system may further receive a second image from the image sensor; determine, from the second image and the digital model of surgical tooling equipment, a third position of the surgical tooling equipment within the second image; and display the cursor of the graphical user interface at a fourth position associated with the third position. The system may further receive user input that indicates a selection while coordinates of the at least one icon include the fourth position. For example, the selection may indicate a selection of the icon. The system may further determine that the user input that indicates the selection while the coordinates of the at least one icon include the fourth position; and in response to determining that the user input that indicates the selection, change data displayed by the graphical user interface. For example, image data of the graphical user interface may be changed in association with a next step of a workflow associated with a surgery. As another example, at least a portion of the first image, the second image, or the third image may be stored in response to determining that the user input that indicates the selection. Image data may be stored via a memory medium.

The present disclosure may further include a non-transient computer-readable memory device with instructions that, when executed by a processor of a system, cause the system to perform the above steps. The present disclosure further includes a system or a non-transient computer-readable memory device as described above with one or more of the following additional features, which may be used in combination with one another unless clearly mutually exclusive: i) as the processor executes the instructions, the system may be further able to display a graphical user interface that includes at least one icon via a display; ii) as the processor executes the instructions, the system may be further able to receive a first image from an image sensor; iii) as the processor executes the instructions, the system may be further able to determine, from the first image and a digital model of surgical tooling equipment, a first position of the surgical tooling equipment within the first image; iv) as the processor executes the instructions, the system may be further able to display a cursor of the graphical user interface at a second position associated with the first position; v) as the processor executes the instructions, the system may be further able to receive a second image from the image sensor; vi) as the processor executes the instructions, the system may be further able to determine, from the second image and the digital model of surgical tooling equipment, a third position of the surgical tooling equipment within the second image; vii) as the processor executes the instructions, the system may be further able to display the cursor of the graphical user interface at a fourth position associated with the third position; viii) as the processor executes the instructions, the system may be further able to receive user input that indicates a selection while coordinates of the at least one icon include the fourth position; ix) as the processor executes the instructions, the system may be further able to determine that the user input that indicates the selection while the coordinates of the at least one icon include the fourth position; x) as the processor executes the instructions, the system may be further able to, in response to determining that the user input that indicates the selection, change data displayed by the graphical user interface; xi) when the system receives the user input that indicates the selection, the system may be further able to receive a third image from the image sensor; xii) when the system determines that the user input that indicates the selection, the system may be further able to determine, based at least on the second and third images and the digital model, a change in a number of pixels associated with the surgical tooling equipment that indicates a change in distance of the surgical tooling equipment to the image sensor; xiii) when the system receives the user input that indicates the selection, the system may be further able to receive multiple of images from the image sensor; xv) when the system determines that the user input that indicates the selection, the system may be further able to determine, based at least on the digital model and the multiple images, a pattern of movement associated with movement of the surgical tooling equipment that indicates the selection; xvi) as the processor executes the instructions, the system may be further able to determine, based at least on the multiple images, the pattern of movement associated with the movement of the surgical tooling equipment that indicates the selection; xvii) as the processor executes the instructions, the system may be further able to store, via the memory medium, at least a portion of the first image, the second image, or a third image in response to determining that the user input that indicates the selection; and xviii) as the processor executes the instructions, the system may be further able to advance a workflow associated with a surgery to a next step of the workflow.

Any of the above systems may be able to perform any of the above methods and any of the above non-transient computer-readable memory devices may be able to cause a system to perform any of the above methods. Any of the above methods may be implemented on any of the above systems or using any of the above non-transient computer-readable memory devices.

It is to be understood that both the foregoing general description and the following detailed description are examples and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTION OF THE DRAWINGS

For a more complete understanding of the present disclosure and its features and advantages, reference is now made to the following description, taken in conjunction with the accompanying drawings, which are not drawn to scale, and in which:
FIG. 1A illustrates an example of a system;
FIG. 1B illustrates an example of a microscope integrated display and examples of surgical tooling equipment;
FIG. 2 illustrates an example of a computer system;
FIGs. 3A and 3B illustrate examples of a graphical user interface;
FIGs. 4A and 4B illustrate examples of registration areas;
FIG. 4C illustrates an example registering a movement pattern;
FIG. 4D illustrates an example receiving a movement pattern;
FIG. 4E illustrates another example receiving a movement pattern;
FIG. 5 illustrates an example of a method utilizing surgical tooling equipment with a graphical user interface; and
FIG. 6 illustrates another example of a method utilizing surgical tooling equipment with a graphical user interface.

### DETAILED DESCRIPTION

In the following description, details are set forth by way of example to facilitate discussion of the disclosed subject matter. It should be apparent to a person of ordinary skill in the field, however, that the disclosed embodiments are examples and not exhaustive of all possible embodiments.

As used herein, a reference numeral refers to a class or type of entity, and any letter following such reference numeral refers to a specific instance of a particular entity of that class or type. Thus, for example, a hypothetical entity referenced by '12A' may refer to a particular instance of a particular class/type, and the reference '12' may refer to a collection of instances belonging to that particular class/type or any one instance of that class/type in general.

A surgeon may be in a sterile surgical environment. The surgeon may use his or her surgical tooling equipment to control and/or direct a graphical user interface (GUI). The GUI may be utilized to control a workflow associated with a surgery. In utilizing surgical tooling equipment to control and/or direct a GUI, a device may determine one or more shapes of surgical tooling equipment. For example, one or more cameras may provide one or more images to the device. The device may determine the surgical tooling equipment from the one or more images from the one or more cameras. The device may track one or more movements of the surgical tooling equipment. For example, the device may track one or more movements of the surgical tooling equipment based at least on the one or more images from the one or more cameras.

The one or more movements of the surgical tooling equipment that are tracked may be utilized in interacting with a GUI. In one example, the GUI may be displayed via a microscope integrated display (MID). In another example, the GUI may be displayed via a display. A surgeon may view and/or interact with the GUI via the MID. The surgeon and/or other surgical personnel may interact with the GUI via the display. The GUI may be overlaid to the surgeon's current area of interest. For example, the GUI may overlay the surgeon's current area of interest so the surgeon may visualize the GUI without looking away from surgeon's current area of interest. For example, the GUI may overlay a live scene. Motion-based object tracking may be utilized in interacting with the GUI. For example, utilizing motion-based object tracking and/or object recognition, surgical tooling equipment may be utilized as a pointing device in interacting with the GUI. Examples of a pointing device may be or include one or more of a mouse, a trackpad, and a trackball, among others.

Surgical tooling equipment may be registered with a system to be utilized in association with the GUI. For example, surgical tooling equipment may be registered with the system to be utilized as of a pointing device to be utilized in association with the GUI. Registering the surgical tooling equipment to be utilized in association with the GUI may include the system receiving one or more images of the surgical tooling equipment and determining one or more shapes and/or one or more curves of the surgical tooling equipment that may be utilized in identifying the surgical tooling equipment. For example, one or more machine learning processes and/or one or more machine learning methods may be utilized in determining one or more shapes and/or one or more curves of the surgical tooling equipment that may be utilized in identifying the surgical tooling equipment. The one or more machine learning processes and/or one or more machine learning methods may produce and/or determine a digital model of the surgical tooling equipment. For example, the digital model may be utilized in inferring one or more positions of the surgical tooling equipment in associated utilization with the GUI.

One or more movements of the surgical tooling equipment may be utilized in determining a pointer "click". For example, one or more movements of the surgical tooling equipment may be utilized as a mouse click. The pointer "click" may indicate a selection of one or more items displayed via the GUI. After the surgical tooling equipment is registered with the system, One or more movements of the surgical tooling equipment may be determined and/or identified as a pointer "click". In one example, a first movement may be utilized as a left mouse button selection (e.g. "click"). In a second example, a second movement may be utilized as a right mouse button selection (e.g. "click"). In a third example, a third movement may be utilized as a left mouse button hold selection (e.g. holding down a left mouse button). In another example, a fourth movement may be utilized as a left mouse button release selection (e.g. releasing a left mouse button). One or more motion-based object tracking processes and/or one or more motion-based object tracking methods may be utilized. For example, the one or more motion-based object tracking processes and/or one or more motion-based object tracking methods may utilize one or more of background subtraction, frame difference, and optical flow, among others, to track surgical tooling equipment.

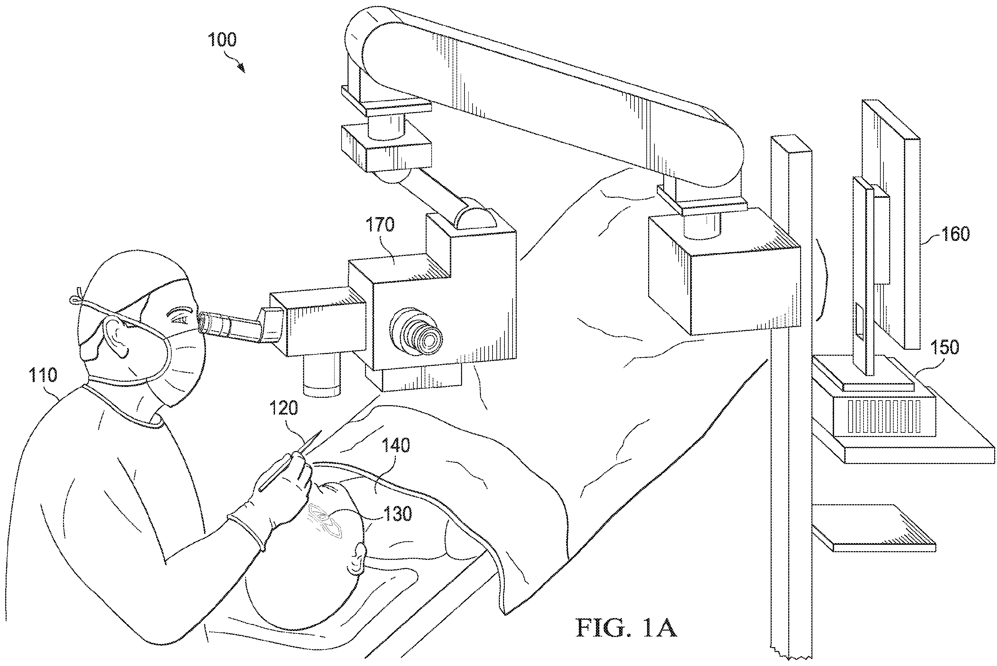

Turning now to FIG. 1A, an example of a system is illustrated. As shown, a surgeon 110 may utilize surgical tooling equipment 120. In one example, surgeon 110 may utilize surgical tooling equipment 120 in a surgery involving a patient portion 130 of a patient 140. For example, surgeon 110 may utilize surgical tooling equipment 120 in interacting with and/or utilizing a system 100. For example, system 100 may be or include an ophthalmic surgical tool tracking system. As illustrated, system 100 may include a computing device 150, a display 160, and a MID 170.

Computing device 150 may receive image frames captured by one or more image sensors. For example, computing device 150 may perform various image processing on the one or more image frames. Computing device 150 may perform image analysis on the one or more image frames to identify and/or extract one or more images of surgical tooling equipment 120 from the one or more image frames. Computing device 150 may generate a GUI, which may overlay the one or more image frames. For example, the GUI may include one or more indicators and/or one or more icons, among others. The one or more indicators may include surgical data, such as one or more positions and/or one or more orientations. The GUI may be displayed by display 160 and/or MID 170 to surgeon 110 and/or other medical personnel.

Computing device 150, display 160, and MID 170 may be implemented in separate housings communicatively coupled to one another or within a common console or housing. A user interface may be associated with one or more of computing device 150, display 160, and MID 170, among others. For example, a user interface may include one or more of a keyboard, a mouse, a joystick, a touchscreen, an eye tracking device, a speech recognition device, a gesture control module, dials, and/or buttons, among other input devices. A user (e.g., surgeon 110 and/or other medical personnel) may enter desired instructions and/or parameters via the user interface. For example, the user interface may be utilized in controlling one or more of computing device 150, display 160, and MID 170, among others.

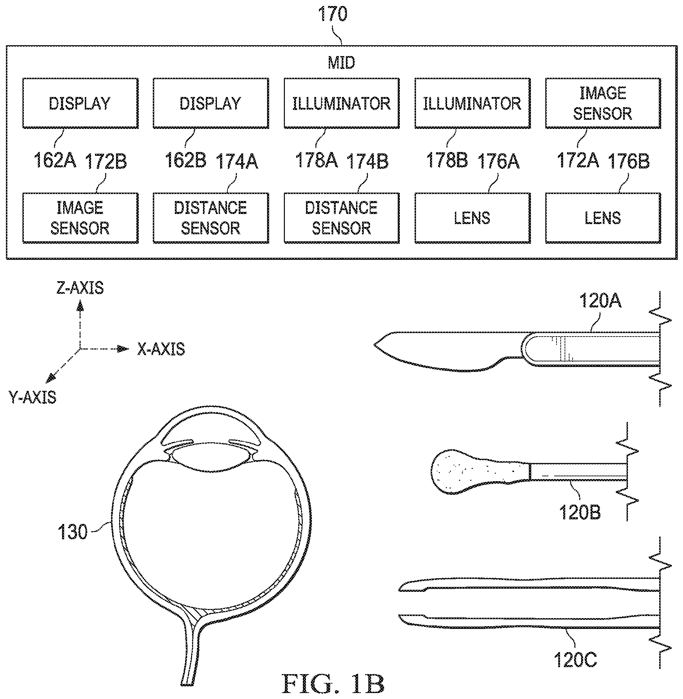

Turning now to FIG. 1B, an example of a microscope integrated display and examples of surgical tooling equipment are illustrated. As shown, surgical tooling equipment 120A may be or include a scalpel. As illustrated, surgical tooling equipment 120B may be or include a Q-tip. As shown, surgical tooling equipment 120C may be or include tweezers. Other surgical tooling equipment that is not specifically illustrated may be utilized with one or more systems, one or more processes, and/or one or more methods described herein.

As an example, surgical tooling equipment 120 may be marked with one or more patterns. The one or more patterns may be utilized in identifying surgical tooling equipment 120. The one or more patterns may include one or more of a hash pattern, a stripe pattern, and a fractal pattern, among others. As another example, surgical tooling equipment 120 may be marked with a dye and/or a paint. The dye and/or the paint may reflect one or more of visible light, infrared light, and ultraviolet light, among others. In one example, an illuminator 178 may provide ultraviolet light, and image sensor 172 may receive the ultraviolet light reflected from surgical tooling equipment 120. Computer system 150 may receive image data, based at least on the ultraviolet light reflected from surgical tooling equipment 120, from image sensor 172 and may utilize the image data, based at least on the ultraviolet light reflected from surgical tooling equipment 120, to identify surgical tooling equipment 120 from other image data provided by image sensor 172. In another example, an illuminator 178 may provide infrared light, and image sensor 172 may receive the infrared light reflected from surgical tooling equipment 120. Computer system 150 may receive image data, based at least on the infrared light reflected from surgical tooling equipment 120, from image sensor 172 and may utilize the image data, based at least on the infrared light reflected from surgical tooling equipment 120, to identify surgical tooling equipment 120 from other image data provided by image sensor 172.

As illustrated, MID 170 may include displays 162A and 162B. For example, surgeon 110 may look into multiple eye pieces, and displays 162A and 162B may display information to surgeon 110. Although MID 170 is shown with multiple displays, MID 170 may include a single display 162. For example, MID 170 may be implemented with one or more displays 162. As shown, MID 170 may include image sensors 172A and 172B. In one example, image sensors 172A and 172B may acquire images. In a second example, image sensors 172A and 172B may include cameras. In another example, an image sensor 172 may acquire images via one or more of visible light, infrared light, and ultraviolet light, among others. One or more image sensors 172A and 172B may provide data of images to computing device 150. Although MID 170 is shown with multiple image sensors, MID 170 may include a single image sensor 172. For example, MID 170 may be implemented with one or more image sensors 172.

As illustrated, MID 170 may include distance sensors 174A and 174. For example, a distance sensor 174 may determine a distance to surgical tooling equipment 120. Distance sensor 174 may determine a distance associated with a Z-axis. Although MID 170 is shown with multiple image sensors, MID 170 may include a single distance sensor 174. In one example, MID 170 may be implemented with one or more distance sensors 174. In another example, MID 170 may be implemented with no distance sensor. As shown, MID 170 may include lenses 176A and 176B. Although MID 170 is shown with multiple lenses 176A and 176B, MID 170 may include a single lens 176. For example, MID 170 may be implemented with one or more lenses 176. As illustrated, MID 170 may include illuminators 178A and 178B. For example, an illuminator 178 may provide and/or produce one or more of visible light, infrared light, and ultraviolet light, among others. Although MID 170 is shown with multiple illuminators, MID 170 may include a single illuminator 178. For example, MID 170 may be implemented with one or more illuminators 178.

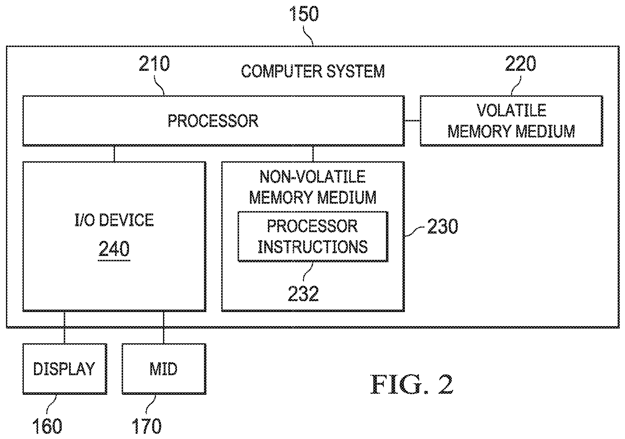

Turning now to FIG. 2, an example of a computer system is illustrated. As shown, computer system 150 may include a processor 210, a volatile memory medium 220, a non-volatile memory medium 230, and an input/output (I/O) device 240,. As illustrated, volatile memory medium 220, non-volatile memory medium 230, and I/O device 240 may be communicatively coupled to processor 210.

The term "memory medium" may mean a "memory", a "storage device", a "memory device", a "computer-readable medium", and/or a "tangible computer readable storage medium". For example, a memory medium may include, without limitation, storage media such as a direct access storage device, including a hard disk drive, a sequential access storage device, such as a tape disk drive, compact disk (CD), random access memory (RAM), read-only memory (ROM), CD-ROM, digital versatile disc (DVD), electrically erasable programmable read-only memory (EEPROM), flash memory, non-transitory media, and/or one or more combinations of the foregoing. As shown, non-volatile memory medium 230 may include processor instructions 232. Processor instructions 232 may be executed by processor. In one example, one or more portions of processor instructions 232 may be executed via non-volatile memory medium 230. In another example, one or more portions of processor instructions 232 may be executed via volatile memory medium 220. One or more portions of processor instructions 232 may be transferred to volatile memory medium 220.

Processor 210 may execute processor instructions 232 in implementing one or more systems, one or more flow charts, one or more processes, and/or one or more methods described herein. For example, processor instructions 232 may be configured, coded, and/or encoded with instructions in accordance with one or more systems, one or more flowcharts, one or more methods, and/or one or more processes described herein. One or more of a storage medium and a memory medium may be a software product, a program product, and/or an article of manufacture. For example, the software product, the program product, and/or the article of manufacture may be configured, coded, and/or encoded with instructions, executable by a processor, in accordance with one or more flowcharts, one or more methods, and/or one or more processes described herein.

Processor 210 may include any suitable system, device, or apparatus operable to interpret and execute program instructions, process data, or both stored in a memory medium and/or received via a network. Processor 210 further may include one or more microprocessors, microcontrollers, digital signal processors (DSPs), application specific integrated circuits (ASICs), or other circuitry configured to interpret and execute program instructions, process data, or both.

I/O device 240 may include any instrumentality or instrumentalities, which allow, permit, and/or enable a user to interact with computer system 150 and its associated components by facilitating input from a user and output to a user. Facilitating input from a user may allow the user to manipulate and/or control computer system 150, and facilitating output to a user may allow computer system 150 to indicate effects of the user's manipulation and/or control. For example, I/O device 240 may allow a user to input data, instructions, or both into computer system 150, and otherwise manipulate and/or control computer system 150 and its associated components. I/O devices may include user interface devices, such as a keyboard, a mouse, a touch screen, a joystick, a handheld lens, a tool tracking device, a coordinate input device, or any other I/O device suitable to be used with a system, such as system 100.

I/O device 240 may include one or more busses, one or more serial devices, and/or one or more network interfaces, among others, that may facilitate and/or permit processor 210 to implement one or more systems, processes, and/or methods described herein. In one example, I/O device 240 may include a storage interface that may facilitate and/or permit processor 210 to communicate with an external storage. The storage interface may include one or more of a universal serial bus (USB) interface, a SATA (Serial ATA) interface, a PATA (Parallel ATA) interface, and a small computer system interface (SCSI), among others. In a second example, I/O device 240 may include a network interface that may facilitate and/or permit processor 210 to communicate with a network. I/O device 240 may include one or more of a wireless network interface and a wired network interface. In a third example, I/O device 240 may include one or more of a peripheral component interconnect (PCI) interface, a PCI Express (PCIe) interface, a serial peripheral interconnect (SPI) interface, and an inter-integrated circuit (I²C) interface, among others. In another example, I/O device 240 may facilitate and/or permit processor 210 to communicate data with one or more of display 160 and MID 170, among others.

As shown, I/O device 240 may be communicatively coupled to display 160 and MID 170. For example, computer system 150 may be communicatively coupled to display 160 and MID 170 via I/O device 240. I/O device 240 may facilitate and/or permit processor 210 to communicate data with one or more elements of MID 170. In one example, I/O device 240 may facilitate and/or permit processor 210 to communicate data with one or more of an image sensor 172, a distance sensor 174, and a display 162, among others. In another example, I/O device 240 may facilitate and/or permit processor 210 to control one or more of an image sensors 172, a distance sensor 174, an illuminator 178, and a display 162, among others.

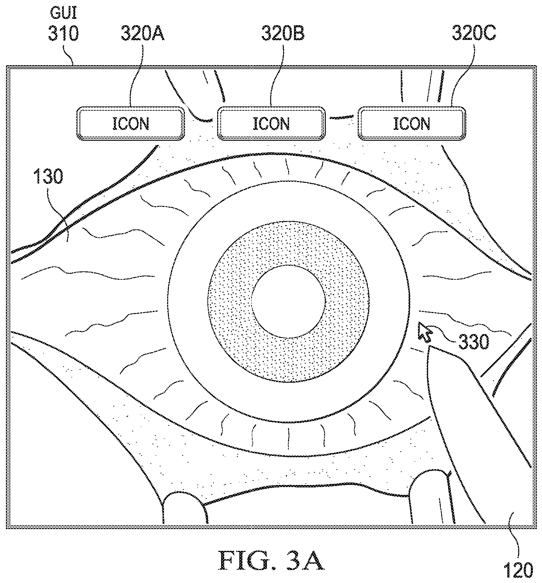

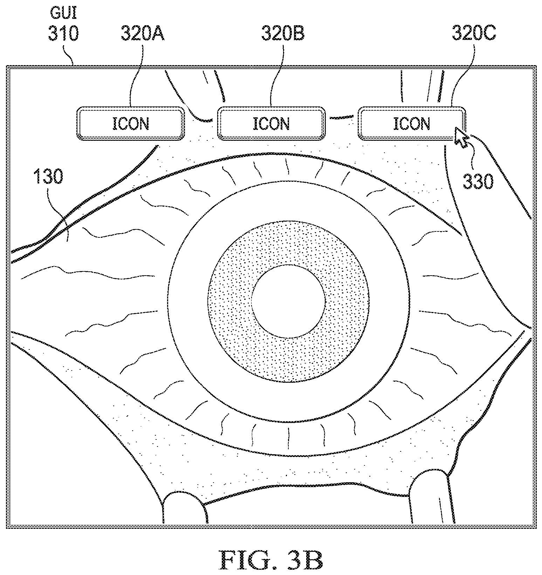

Turning now to FIGs. 3A and 3B, examples of a graphical user interface are illustrated. As shown, a GUI 310 may include icons 320A-320C. For example, GUI 310 and/or icons 320A-320C may be overlaid on an image acquired via an image sensor 172. As illustrated, GUI 310 may display a cursor 330. For example, system 100 may track movements of surgical tooling equipment 120 and display cursor 330 based one or more movements and/or one or more positions of surgical tooling equipment 120. System 100 may track movements of surgical tooling equipment 120. For example, system 100 may track one or more movements and/or one or more positions of surgical tooling equipment 120 to icon 320C.

GUI 310 may be displayed via a display. For example, GUI 310 may be displayed via one or more of displays 160, 162A, and 162B, among others. Surgeon 110 may select icon 320C. In one example, surgeon 110 may select icon 320C via a foot pedal. An actuation of a foot pedal may be utilized as a pointer click (e.g., a mouse click). In another example, surgeon 110 may select icon 320C via one or more movements of surgical tooling equipment 120. The one or more movements of surgical tooling equipment 120 may be utilized as a pointer click (e.g., a mouse click).

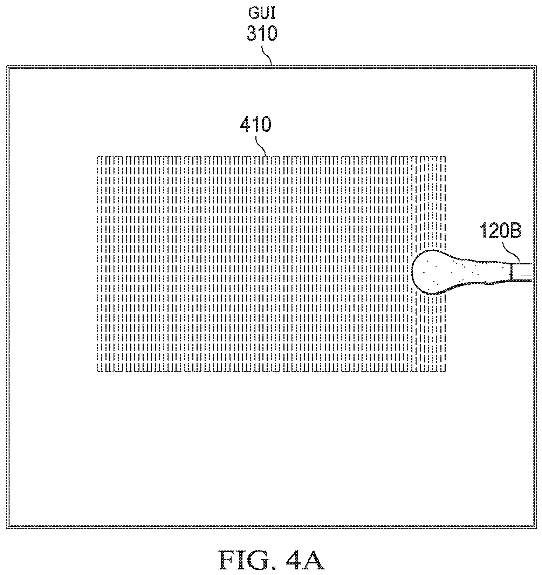

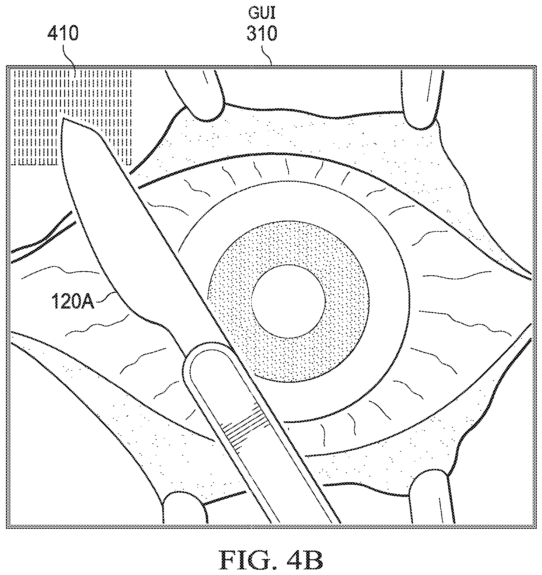

Turning now to FIGs. 4A and 4B, examples of registration areas are illustrated. As shown in FIG. 4A, surgical tooling equipment 120B may be registered via a registration area 410. For example, registration area 410 may be displayed via GUI 310. As illustrated in FIG. 4B, surgical tooling equipment 120A may be registered via registration area 410. For example, via registration area 410 may overlay an acquired image. The acquired image may have been acquired via an image sensor 172A and 172B.

A digital model of surgical tooling equipment 120 may be determined from one or more images from one or more image sensors 172. The digital model of surgical tooling equipment 120 may include a pattern of surgical tooling equipment 120. As an example, the digital model may be utilized in relating image data of surgical tooling equipment 120 within an image acquired via one or more of image sensors 172A and 172B. The digital model may include possible relationships between image data of the surgical tooling equipment within an image acquired via one or more of image sensors 172A and 172B. For example, digital model may include parameters may determine the possible relationships. A learning process and/or method may fit the parameters utilizing training data. In one example, one or more images may be utilized as training data. In another example, registration area 410 may be utilized in associating image data as training data. Determining the digital model of surgical tooling equipment 120 may include training the digital model based at least on the one or more images. The digital model may be discriminative. The digital model may be generative. One or more inference processes and/or one or more methods may utilize the digital model to determine image data of surgical tooling equipment 120 within an image acquired via one or more of image sensors 172A and 172B.

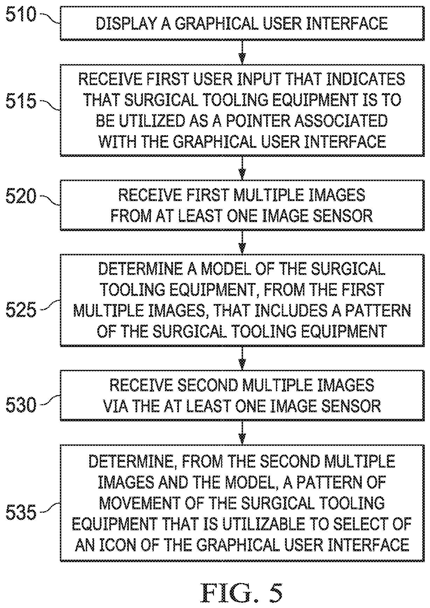

Turning now to FIG. 5, an example of a method utilizing surgical tooling equipment with a graphical user interface is illustrated. At 510, a graphical user interface may be displayed via a display. In one example, GUI 310 may be displayed via display 160. In another example, GUI 310 may be displayed via one or more of display 162A and 162B. At 515, first user input that indicates that surgical tooling equipment is to be utilized as a pointer associated with the graphical user interface may be received. In one example, the first user input may include an actuation of a foot pedal. In a second example, the first user input may include voice input. In another example, the first user input may include an actuation of a GUI icon. Surgeon 110 or other medical personnel may actuate the GUI icon.

At 520, first multiple images from at least one image sensor may be received. For example, first multiple images from one or more of image sensors 172A and 172B may be received. The first multiple images may include image data of the surgical tooling equipment that is to be utilized as the pointer associated with the graphical user interface. At 525, a digital model, that includes a pattern of the surgical tooling equipment, of the surgical tooling equipment, may be determined from the first multiple images. For example, the digital model may be utilized in relating image data of the surgical tooling equipment within an image acquired via one or more of image sensors 172A and 172B. The digital model may include possible relationships between image data of the surgical tooling equipment within an image acquired via one or more of image sensors 172A and 172B. For example, digital model may include parameters may determine the possible relationships. A learning process and/or method may fit the parameters utilizing training data. For example, the first multiple images may be utilized as training data. Determining the digital model of surgical tooling equipment may include training the digital model based at least on the first multiple images. The digital model may be discriminative. The digital model may be generative. An inference process and/or method may utilize the digital model to determine image data of the surgical tooling equipment within an image acquired via one or more of image sensors 172A and 172B.

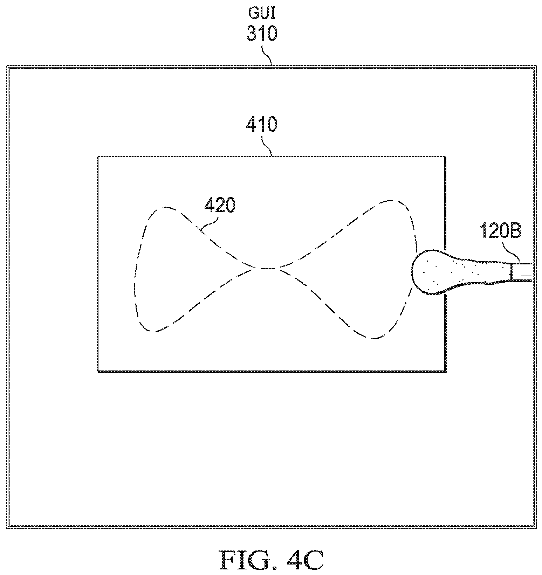

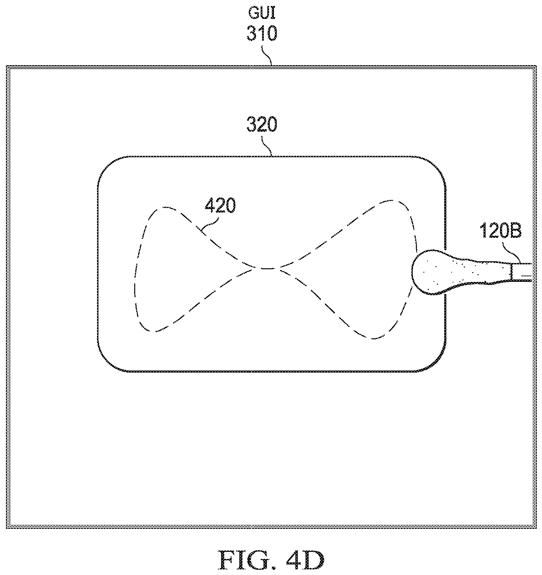

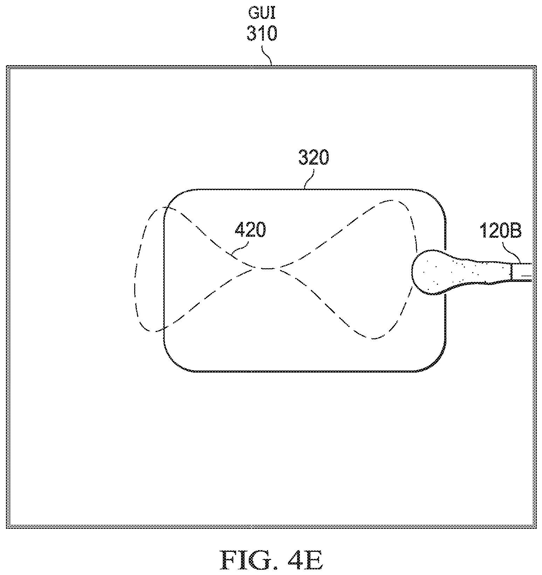

At 530, second multiple images may be received via the at least one image sensor. For example, second multiple images from one or more of image sensors 172A and 172B may be received. The second multiple images may include image data of the surgical tooling equipment. At 535, a pattern of movement of the surgical tooling equipment that is utilizable to select of an icon of the graphical user interface may be determined from the second multiple images and the digital model. For example, a pattern 420, illustrated in FIG. 4C, may be determined from the second multiple images and the digital model. Pattern 420 may be utilized to select an icon 320, as shown in FIG. 4D. Pattern 420 may be utilized to select an icon 320, as shown in FIG. 4E. For example, at least a portion of pattern 420 may overlap icon 420.

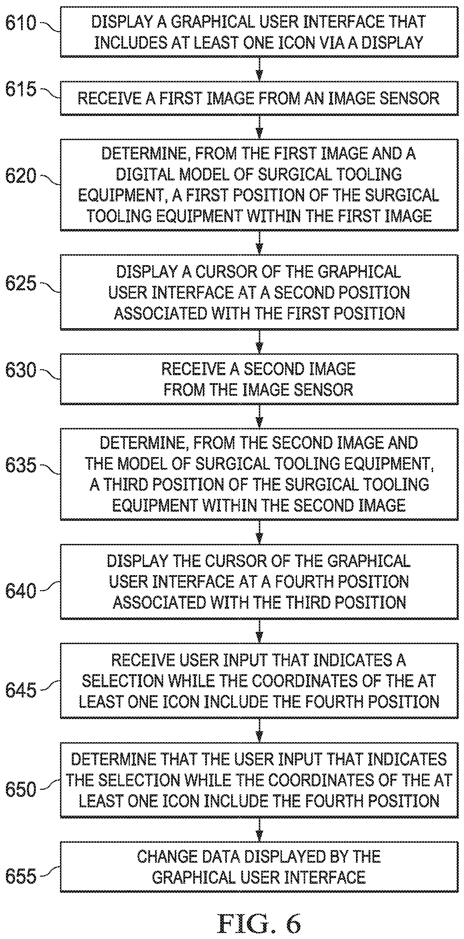

Turning now to FIG. 6, another example of a method utilizing surgical tooling equipment with a graphical user interface is illustrated. At 610, a graphical user interface that includes at least one icon may be displayed via a display. In one example, GUI 310 may be displayed via display 160. In another example, GUI 310 may be displayed via one or more of display 162A and 162B. At 615, a first image from an image sensor may be received. For example, a first image from image sensor 172 may be received. At 620, a first position of the surgical tooling equipment within the first image may be determined from the first image and a digital model of surgical tooling equipment. For example, first position of surgical tooling equipment 120, shown in FIG. 3A, may be determined from the first image and a digital model of surgical tooling equipment. As an example, the digital model may be or include the digital model determined via method element 525 of FIG. 5. As another example, the digital model may be retrieved from a memory medium. A memory medium may store one or more digital models of surgical tooling equipment. For example, the memory medium may store a library that includes one or more digital models of surgical tooling equipment.

At 625, a cursor of the graphical user interface at a second position associated with the first position may be displayed. For example, cursor 330 of GUI 310, shown in FIG. 3A, may be displayed at a second position associated with the first position. At 630, a second image from the image sensor may be received. For example, a second image from image sensor 172 may be received. At 635, a third position of the surgical tooling equipment within the second image may be determined from the second image and the digital model of surgical tooling equipment. For example, a third position of surgical tooling equipment 120, shown in FIG. 3B, may be determined from the second image and the digital model of surgical tooling equipment. At 640, the cursor of the graphical user interface may be displayed at a fourth position associated with the third position. For example, cursor 330 of GUI 310, shown in FIG. 3B, may be displayed at a fourth position associated with the third position.

At 645, user input that indicates a selection, while coordinates of the at least one icon include the fourth position, may be received. In one example, the user input may include an actuation of a foot pedal. Surgeon 110 may actuate the foot pedal as the user input that indicates the selection. In a second example, the user input may include a movement pattern. The user input may include movement pattern 420 shown in FIGs. 4D and 4E. The movement pattern may be approximate to movement pattern 420 shown in FIGs. 4D and 4E. Other movement patterns may be configured and/or utilized. In a third example, the user input may include a change in a number of pixels associated with the surgical tooling equipment that indicates a change in distance of the surgical tooling equipment to the image sensor. A number of pixels associated with the surgical tooling equipment may increase if the surgical tooling equipment is brought closer to the image sensor. As an example, receive a third image from the image sensor from the image sensor may be received, and a change in a number of pixels associated with the surgical tooling equipment that indicates a change in distance of the surgical tooling equipment to the image sensor may be determined based at least on the second and third images and the digital model.

At 650, it may be determined that the user input that indicates the selection while the coordinates of the at least one icon include the fourth position. For example, it may be determined that the user input that indicates a selection of icon 320. At 655, data displayed by the graphical user interface may be changed. For example, image data of GUI 310 may be changed. Changing the data displayed by the graphical user interface may be performed in response to determining that the user input that indicates the selection. As an example, a workflow associated with a surgery may proceed to a next step of the workflow. Image data of GUI 310 may be changed in association with the next step of the workflow associated with the surgery. As another example, at least a portion of the first image, the second image, or the third image may be stored in response to determining that the user input that indicates the selection. Image data may be stored via a memory medium.

One or more of the method and/or process elements and/or one or more portions of a method and/or processor elements may be performed in varying orders, may be repeated, or may be omitted. Furthermore, additional, supplementary, and/or duplicated method and/or process elements may be implemented, instantiated, and/or performed as desired. Moreover, one or more of system elements may be omitted and/or additional system elements may be added as desired.

A memory medium may be and/or may include an article of manufacture. For example, the article of manufacture may include and/or may be a software product and/or a program product. The memory medium may be coded and/or encoded with processor-executable instructions in accordance with one or more flowcharts, systems, methods, and/or processes described herein to produce the article of manufacture.

The above disclosed subject matter is to be considered illustrative, and not restrictive, and the appended claims are intended to cover all such modifications, enhancements, and other implementations which fall within their scope. Thus, to the maximum extent allowed by law, the scope of the present disclosure is to be determined by the broadest permissible interpretation of the following claims, and shall not be restricted or limited by the foregoing detailed description.

## Claims

1. A system (100), comprising:
a surgical tooling equipment (120);
a display (160, 162A-B);
at least one processor (210); and
a memory medium (230) that is coupled to the at least one processor (210), the memory medium comprising a digital model of said surgical tooling equipment,
wherein the memory medium includes instructions, which when executed by the at least one processor (210), cause the system (100) to:
display a graphical user interface (310) that includes at least one icon (320A-C) via the display (160, 162A-B);
receive a first image from an image sensor (172A-B);
determine, from the first image and the digital model of the surgical tooling equipment (120), a first position of the surgical tooling equipment (120) within the first image;
display a cursor (330) of the graphical user interface (310) at a second position associated with the first position;
receive a second image from the image sensor (172A-B);
determine, from the second image and the digital model of the surgical tooling equipment (120), a third position of the surgical tooling equipment (120) within the second image;
display the cursor of the graphical user interface (310) at a fourth position associated with the third position;
receive user input that indicates a selection while coordinates of the at least one icon (320A-C) include the fourth position;
determine that the user input indicates the selection while the coordinates of the at least one icon (320A-C) include the fourth position; and
in response to determining that the user input indicates the selection, change data displayed by the graphical user interface (310),
wherein the system (100) is configured to track movements of the surgical tooling equipment (120) and to display the cursor (330) based on one or more movements of the surgical tooling equipment (120),
wherein, to receive the user input that indicates the selection, the instructions further cause the system (100) to receive a third image from the image sensor (172A-B); and
wherein, to determine that the user input indicates the selection, the instructions further cause the system (100) to determine, based at least on the second and third images and the digital model, a change in a number of pixels associated with the surgical tooling equipment (120) that indicates a change in distance of the surgical tooling equipment (120) to the image sensor (172A-B).

2. The system (100) of claim 1, wherein the instructions further cause the system (100) to:
store, via the memory medium (230), at least a portion of the first image, the second image, or a third image in response to determining that the user input indicates the selection.

3. The system (100) of claim 1, wherein the instructions further cause the system (100) to:
advance a workflow associated with a surgery to a next step of the workflow.

4. The system (100) of claim 1, further comprising:
a microscope integrated display (170);
wherein the microscope integrated display (170) includes the display (162A-B).

## Patentansprüche

1. System (100), umfassend:
eine chirurgische Werkzeugausrüstung (120);
eine Anzeige (160, 162A-B);
wenigstens einen Prozessor (210); und
ein Speichermedium (230), das an den wenigstens einen Prozessor (210) gekoppelt ist, wobei das Speichermedium ein digitales Modell der chirurgischen Werkzeugausrüstung umfasst,
wobei das Speichermedium Anweisungen beinhaltet, die, wenn sie von dem wenigstens einen Prozessor (210) ausgeführt werden, das System (100) hierzu veranlassen: Anzeigen einer grafischen Benutzerschnittstelle (310), die wenigstens ein Symbol (320A-C) beinhaltet, über die Anzeige (160, 162A-B);
Empfangen eines ersten Bildes von einem Bildsensor (172AB);
Bestimmen, anhand des ersten Bildes und des digitalen Modells der chirurgischen Werkzeugausrüstung (120), einer ersten Position der chirurgischen Werkzeugausrüstung (120) in dem ersten Bild;
Anzeigen eines Cursors (330) der grafischen Benutzerschnittstelle (310) an einer zweiten Position, die mit der ersten Position verknüpft ist;
Empfangen eines zweiten Bildes von dem Bildsensor (172AB);
Bestimmen, anhand des zweiten Bildes und des digitalen Modells der chirurgischen Werkzeugausrüstung (120), einer dritten Position der chirurgischen Werkzeugausrüstung (120) in dem zweiten Bild;
Anzeigen des Cursors der grafischen Benutzerschnittstelle (310) an einer vierten Position, die mit der dritten Position verknüpft ist;
Empfangen einer Benutzereingabe, die eine Auswahl angibt, während Koordinaten des wenigstens einen Symbols (320A-C) die vierte Position beinhalten;
Bestimmen, dass die Benutzereingabe die Auswahl angibt, während die Koordinaten des wenigstens einen Symbols (320A-C) die vierte Position beinhalten; und
in Reaktion auf das Bestimmen, dass die Benutzereingabe die Auswahl angibt, Ändern von Daten, die durch die grafische Benutzerschnittstelle (310) angezeigt werden, wobei das System (100) dazu ausgelegt ist, Bewegungen der chirurgischen Werkzeugausrüstung (120) zu verfolgen und den Cursor (330) basierend auf einer oder mehreren Bewegungen der chirurgischen Werkzeugausrüstung (120) anzuzeigen,
wobei, um die Benutzereingabe zu empfangen, welche die Auswahl angibt, die Anweisungen das System (100) ferner veranlassen, ein drittes Bild von dem Bildsensor (172AB) zu empfangen; und
wobei, um zu bestimmen, dass die Benutzereingabe die Auswahl angibt, die Anweisungen das System (100) ferner veranlassen, basierend wenigstens auf dem zweiten und dem dritten Bild und dem digitalen Modell, eine Änderung hinsichtlich einer mit der chirurgischen Werkzeugausrüstung (120) verknüpften Anzahl von Pixeln zu bestimmen, die eine Änderung hinsichtlich des Abstands der chirurgischen Werkzeugausrüstung (120) zu dem Bildsensor (172A-B) angibt.

2. System (100) nach Anspruch 1, wobei die Anweisungen das System (100) ferner hierzu veranlassen:
Speichern, über das Speichermedium (230), wenigstens eines Teils des ersten Bildes, des zweiten Bildes oder eines dritten Bildes in Reaktion auf das Bestimmen, dass die Benutzereingabe die Auswahl angibt.

3. System (100) nach Anspruch 1, wobei die Anweisungen das System (100) ferner hierzu veranlassen:
Vorrücken eines Arbeitsablaufs, der mit einer Operation verknüpft ist, zu einem nächsten Schritt des Arbeitsablaufs.

4. System (100) nach Anspruch 1, ferner umfassend:
eine integrierte Mikroskopanzeige (170);
wobei die integrierte Mikroskopanzeige (170) die Anzeige (162A-B) beinhaltet;

## Revendications

1. Système (100) comprenant :
un équipement d'instrumentation chirurgicale (120) ;
un affichage (160, 162A-B) ;
au moins un processeur (210) ; et
un support de mémoire (230) couplé à l'au moins un processeur (210), le support de mémoire comprenant un modèle numérique dudit équipement d'instrumentation chirurgicale,
le support de mémoire comportant des instructions qui, lorsqu'elles sont exécutées par l'au moins un processeur (210), amènent le système (100) à :
afficher, via l'affichage (160, 162A-B), une interface utilisateur graphique (310) qui comporte au moins une icône (320A-C) ;
recevoir une première image provenant d'un capteur d'images (172A-B) ;
déterminer, à partir de la première image et du modèle numérique de l'équipement d'instrumentation chirurgicale (120), une première position de l'équipement d'instrumentation chirurgicale (120) au sein de la première image ;
afficher un curseur (330) de l'interface utilisateur graphique (310) à une deuxième position associée à la première position ;
recevoir une deuxième image provenant du capteur d'images (172A-B) ;
déterminer, à partir de la deuxième image et du modèle numérique de l'équipement d'instrumentation chirurgicale (120), une troisième position de l'équipement d'instrumentation chirurgicale (120) au sein de la deuxième image ;
afficher le curseur de l'interface utilisateur graphique (310) à une quatrième position associée à la troisième position ;
recevoir une entrée utilisateur qui indique une sélection lorsque des coordonnées de l'au moins une icône (320A-C) comportent la quatrième position ;
déterminer que l'entrée utilisateur indique la sélection lorsque les coordonnées de l'au moins une icône (320A-C) comportent la quatrième position ; et
en réponse à la détermination que l'entrée utilisateur indique la sélection, modifier des données affichées par l'interface utilisateur graphique (310),
le système (100) étant configuré pour suivre des mouvements de l'équipement d'instrumentation chirurgicale (120) et pour afficher le curseur (330) sur la base d'un ou de plusieurs mouvements de l'équipement d'instrumentation chirurgicale (120),
pour recevoir l'entrée utilisateur qui indique la sélection, les instructions amenant en outre le système (100) à recevoir une troisième image provenant du capteur d'images (172A-B) ; et
pour déterminer que l'entrée utilisateur indique la sélection, les instructions amenant en outre le système (100) à déterminer, sur la base au moins des deuxième et troisième images et du modèle numérique, une variation d'un nombre de pixels associé à l'équipement d'instrumentation chirurgicale (120) qui indique une variation de distance de l'équipement d'instrumentation chirurgicale (120) par rapport au capteur d'images (172A-B).

2. Système (100) selon la revendication 1, dans lequel les instructions amènent en outre le système (100) à : stocker, via le support de mémoire (230), au moins une partie de la première image, de la deuxième image, ou d'une troisième image en réponse à la détermination que l'entrée utilisateur indique la sélection.

3. Système (100) selon la revendication 1, dans lequel les instructions amènent en outre le système (100) à :
faire progresser un flux de travail associé à une intervention chirurgicale vers une étape suivante du flux de travail.

4. Système (100) selon la revendication 1, comprenant en outre :
un affichage (170) intégré au microscope ;
l'affichage (170) intégré au microscope comportant l'affichage (162A-B).
